# EUROPEAN PATENT SPECIFICATION

(11) **EP 1 971 344 B1**
(45) Date of publication and mention of the grant of the patent: **22.09.2010**
(21) Application number: 07709154.4
(22) Date of filing: 09.01.2007
(51) Int. Cl.: A61K 31/565, A61P 9/00, A61P 9/10, A61K 31/519, A61K 31/57, A61K 31/34, A61K 31/4045

(54) **A METHOD OF TREATING AN ACUTE VASCULAR DISORDER**
VERFAHREN ZUR BEHANDLUNG EINER AKUTEN GEFÄSSERKRANKUNG
PROCÉDÉ DE TRAITEMENT D'UN TROUBLE VASCULAIRE AIGU

(30) Priority: 09.01.2006 EP 06100159; 09.01.2006 US 757027 P
(43) Date of publication of application: 24.09.2008
(73) Proprietor: Pantarhei Bioscience B.V., 3701 CH Zeist (NL)
(72) Inventor: BENNINK, Herman Jan Thijmen Coelingh, NL-3985 MK Werkhoven (NL)
(74) Representative: Swinkels, Bart Willem
(86) International application number: PCT/NL2007/050006
(87) International publication number: WO 2007/081206

(56) References cited:
- WO-A-03/103683

## Description

### TECHNICAL FIELD

The present invention is concerned with the use of a steroid in the manufacture of a pharmaceutical composition for use in a method of treating an acute vascular disorder, e.g. a cardiovascular, cerebrovascular or peripheral vascular disorder, said method comprising ad lib oral administration to the mammal, upon demand, of an effective amount of a steroid.

### BACKGROUND OF THE INVENTION

Vascular disease can affect any part of the arterial system. Smoking, high blood pressure, obesity, high blood cholesterol, and diabetes are risk factors for all types of vascular disease.

Acute cardiovascular disorders include, for example, angina pectoris, myocardial ischemia, myocardial infarction and diseases that arise from thrombotic states in which the coagulation cascade is activated.

Angina pectoris is typically described as a substernal chest discomfort perceived as tightness, heaviness, or pressure, or a burning sensation. Angina pectoris results when myocardial oxygen demand is increased to levels that cannot be met through increased coronary blood flow, usually because of stenotic atherosclerotic lesions in one or more of the epicardial coronary vessels. Accordingly, angina is typically brought on by physical exertion or emotional stress.

Most patients with stable angina can identify specific activities or situations that will predictably elicit the discomfort; walking up an incline or hurrying are common examples. Angina pectoris that has recently progressed or spontaneously increased in severity, frequency, or duration, particularly if accompanied by rest pain, is considered unstable angina. Patients with the recent onset of angina, particularly if it occurs at low levels of activity or at rest, are also included in this category.

Ischemia is a condition in which an organ or a part of the body fails to receive a sufficient blood supply. When an organ is deprived of a blood supply, it is said to be hypoxic. An organ will become hypoxic even when the blood supply temporarily ceases, such as during a surgical procedure or during temporary artery blockage. Ischemia initially leads to a decrease in or loss of contractile activity. When the organ affected is the heart, this condition is known as myocardial ischemia. When myocardial ischemia is of sufficient severity and duration, cell injury can progress to cell death, i.e. myocardial infarction, and subsequently to heart failure, hypertrophy, or congestive heart failure.

Acute cerebrovascular disorders include, for example, migraine, cerebral ischemia, cerebral infarction, cerebral vasospasm and thrombotic stroke.

Migraine is a painful syndrome characterised by unilateral, pulsating headaches, nausea, vomiting, and sensitivity to light and sound. The pathophysiology of migraine headaches involves vasoconstriction (the closing or tightening of arteries, which reduces blood flow) and vasodilatation (the opening of the vessels to increase blood flow). It appears that a variety of stimuli, such as intense light, noise, anxiety, exertion, cold, heat, hormones, food additives and certain foods, result in constriction of extracranial vessels. The vasoconstriction is followed by a sequential or reflex powerful vasodilatation, which subsequently spreads to intracranial vessels. It is during this phase that a patient feels an intense, throbbing headache.

Cerebral infarction is a severe cerebral dysfunction induced by cerebral ischemia or hypoxemia. Conditions which may cause cerebral ischemia include cerebral thrombosis and cerebral embolism.

The term "cerebral vasospasm" is commonly used to refer to both the clinical picture of delayed onset of ischemic neurological deficits associated with aneurysmal subarachnoid haemorrhage ("symptomatic vasospasm") and the narrowing of cerebral vessels documented by angiography or other studies ("angiographic or arterial vasospasm"). Arterial vasospasm typically appears 3 to 4 days after rupture and reaches a peak in incidence and severity at 7- 10 days. The incidence and time course of symptomatic vasospasm parallels that of arterial vasospasm. However, while 40% to 70% of patients have evidence of arterial narrowing (angiography or Doppler ultrasound), only 20% to 30% develop the clinical syndrome. The most important factors in determining the clinical effect of vasospasm are the severity and extent of vessel narrowing. Symptomatic vasospasm typically begins 4-5 days after the haemorrhage and is characterized by the insidious onset of confusion and a decreasing level of consciousness. When the arterial narrowing is severe, these symptoms may progress to focal neurological deficits, infarction, coma and death.

Acute peripheral vascular disorders include, for example, occlusive peripheral arterial disease, Buerger's disease, erectile dysfunction (impotence) and functional peripheral arterial diseases, such as, Raynaud's disease, Raynaud's phenomenon and acrocyanosis.

Most people with occlusive peripheral arterial disease have atherosclerosis, a disease process in which fatty material accumulates under the lining of the arterial wall, gradually narrowing the artery. However, a partial or complete occlusion of an artery can result from other causes, such as a blood clot. When an artery narrows, the parts of the body it serves may not receive enough blood, resulting in a decrease in oxygen supply (ischemia). Depending on the site of the body affected, obstruction of a peripheral artery may produce diverse symptoms, which may sometimes be life-threatening such as obstruction of the abdominal aorta or the renal arteries. With a narrowing leg artery, the first symptom is a painful, aching, cramping, or tired feeling in leg muscles during physical activity; this feeling is called intermittent claudication. Muscles hurt when the person walks, and the pain comes on faster and is more severe when the person walks quickly or uphill. Most commonly, the pain is in the calf, but it can also be in the foot, thigh, hip, or buttocks, depending on the location of the narrowing. The same kind of pain on exertion is also caused by narrowing of the arteries in the arms. Some relief of symptoms may be obtained by pentoxifylline, calcium antagonists or aspirin. However, beta-blockers which are prescribed to patients with coronary artery obstructions worsen symptoms in patients with leg or arm artery obstructions.

Buerger's disease (thromboangiitis obliterans) is the obstruction of small and medium-sized arteries and veins by inflammation triggered by smoking. Symptoms of reduced blood supply to the arms or legs develop gradually, starting at the fingertips or toes and progressing up the arms or legs, Patients may feel coldness, numbness, tingling, or burning. They often have Raynaud's phenomenon (see below) and experience muscle cramps.

The major cause of erectile dysfunction is vascular; other large pathogenic categories include hormonal disorders, drug use, and neurologic disorders. The major types of vascular problems that can result in erectile dysfunction are atherosclerotic disease of penile arteries, inadequate impedance of venous outflow (venous leaks), or a combination of both. With age and underlying diseases (eg, atherosclerosis, hypertension), dilation of arterial blood vessels and smooth muscle relaxation decrease, diminishing the amount of blood entering the penis. Venous leaks make it difficult for blood to remain in the penis during erection. Diseases that accelerate atherosclerosis (eg, diabetes, smoking, hypertension) increase the prevalence of erectile dysfunction. Sildenafil is a newly released oral drug belonging to the class of phosphodiesterases inhibitors. It promotes erection by potentiating the effect of nitric oxide on vascular smooth muscle, thus increasing blood flow to the penis. It creates more normal erectile response because it works with concomitant sexual arousal. The drug must be taken 30 to 60 minutes before sex and is contraindicated in persons taking nitrates; it causes headaches in about 16% of users.

Raynaud's disease and Raynaud's phenomenon are conditions in which small arteries (arterioles), usually in the fingers and toes, go into spasm, causing the skin to become pale or a patchy red to blue. Spasm of small arteries in the fingers and toes comes on quickly, most often triggered by exposure to cold and may last for minutes or hours. Between 60 and 90 percent of the cases of Raynaud's disease occur in young women. Possible underlying causes of Raynaud's disease and Raynaud's phenomenon include scleroderma, rheumatoid arthritis, atherosclerosis, nerve disorders, decreased thyroid activity, injury, and reactions to certain drugs, such as ergotamine and methysergide. Some people with Raynaud's phenomenon also have migraine headaches, variant angina, and high blood pressure in their lungs (pulmonary hypertension). These associations suggest that the cause of the arterial spasms may be the same in all these disorders. Anything that stimulates the sympathetic nervous system, such as emotion or exposure to cold, can cause arterial spasms. Occasionally, in some patients drugs such as phenoxybenzamine, methyldopa, or pentoxifylline help to relieve symptoms.

Acrocyanosis is a persistent, painless blueness of both hands and, less commonly, the feet, caused by unexplained spasm of the small blood vessels of the skin. The disorder usually occurs in women, not necessarily those with occlusive arterial disease. The fingers or toes and hands or feet are constantly cold and bluish and sweat profusely; they may swell.

It is known in the art that patients suffering from the above mentioned acute cardiovascular, cerebrovascular and peripheral vascular disorders may be treated successfully with vasodilating agents such as nitroglycerin, isosorbide dinitrate, isosorbide mononitrate, minoxidil, papaverine HCL, isoxsuprine HCL, hydralazine, pentoxifylline and sildenafil. However, the use of these vasodilating agents is associated with drawbacks such a slow onset of action and/or undesirable side-effects (e.g. headache, nausea, dizziness, hypotension).

WO 03/103683 describes the use of estetrol and estetrol derivatives for treating or preventing cardiovascular pathologies such as hypercholesterolemia, dyslipidemia, atherosclerosis, arteriosclerosis, xanthomatosis, myocardial infarction, stroke, multiple silent stroke, unstable angina, loss of cognitive function, vascular dementia and Alzheimer disease,

### SUMMARY OF THE INVENTION

The present inventors have unexpectedly found that particular steroids may be employed advantageously in methods for treating the aforementioned acute cardiovascular, cerebrovascular disorders and peripheral vascular disorders that employ ad lib oral administration within 30 minutes after the mammal has started experiencing first symptoms. The steroids that are employed in accordance with the present invention are represented by the following formula 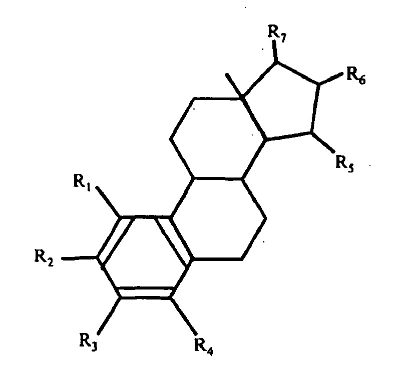 in which formula R₁, R₂, R₃, R₄ independently are a hydrogen atom; a hydroxyl group or an alkoxy group with 1-5 carbon atoms; each of R₅, R₆, R₇ is a hydroxyl group; and no more than 3 of R₁, R₂, R₃, R₄ are hydrogen atoms.

It is known in the art that estrogens, in particular 17β-estradiol, can have a relaxant effect on coronary and cerebral arteries as for example demonstrated *in vitro* by Ospina et al. (Am J Physiol Heart Circ Physiol, 2003 Jul;285(1):H241-50), Salom et al. (J Cereb Blood Flow Metab. 2001 Apr;21(4):422-9) and Kalenic et al. (W V Med J. 2000 Nov-Dec;96(6):617-21). Also it has been observed by Martin et al. (Headache, 2003 Apr; 43(3):309-21) that estrogen add-back therapy in medically oophorectomised females provides a modest benefit in the prevention of migraine headaches. However, pharmacokinetic deficits, such as delayed onset of action and rapid clearance by the liver, combined with a high risk of inducing venous thromboembolism/pulmonary embolism and hepatotoxicity at the required high dose levels have so far prevented the successful treatment of acute cardiovascular, cerebrovascular or peripheral vascular disorders with orally delivered estrogens, e.g. 17β-estradiol.

Surprisingly, it was found that, following oral administration, the steroids according to the present invention exhibit an extremely rapid onset of action. Thus, the present steroids may suitably be (self-)administered orally for instant treatment of an acute vascular disorder.

The high efficacy of the present steroids in the treatment of acute vascular disorders is believed to be associated with the relatively high affinity of these substances for the estrogen receptor a (ERa) as compared to the estrogen receptor β (ERβ).

A typical representative of the present steroids, the natural fetal compound estetrol, interacts with estrogen receptors in a selective manner which is similar to that of so called Selective Estrogen Receptor Modulators (SERM). In view of their SERM-like properties and their high ERa affinity, the steroids of the present invention will be referred to as 'α-SERM'.

### DETAILED DESCRIPTION OF THE INVENTION

Accordingly, the present invention relates to the use of a steroid in the manufacture of a pharmaceutical composition for use in a method of treating an acute vascular disorder in a mammal, said method comprising ad lib oral administration to said mammal, upon demand, of an effective amount of a steroid to said mammal, said steroid being selected from the group consisting of:
substances represented by the following formula 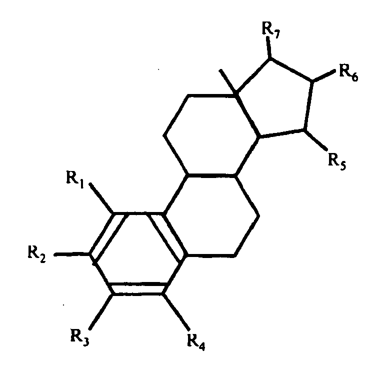 in which formula R₁, R₂, R₃, R₄ independently are a hydrogen atom, a hydroxyl group or an alkoxy group with 1-5 carbon atoms; each of R₅, R₆, R₇ is a hydroxyl group; no more than 3 of R₁, R₂, R₃, R₄ are hydrogen atoms;
precursors capable of liberating a substance according to the aforementioned formula when used in the present method, which precursors are derivatives of the aforementioned steroids wherein the hydrogen atom of at least one of the hydroxyl groups has been substituted by an acyl radical of a hydrocarbon carboxylic, sulfonic or sulfamic acid of 1-25 carbon atoms; tetrahydrofuranyl; tetrahydropyranal; or a straight or branched chain glycosidic residue containing 1-20 glycosidic units per residue; and mixtures of one or more of the aforementioned substances and/or precursors, wherein the steroid is administered within 30 minutes after the mammal has started experiencing first symptoms associated with the acute vascular disorder.

The term "acute vascular disorder" refers to vascular disorders of a non-chronic nature with sudden manifestations of symptoms.

The term "on demand" as used herein means that the steroid is administered as soon as it has been established that the mammal is experiencing, or is about to experience symptoms associated with an acute vascular disorder.

The present method does not encompass the prophylactic treatment of the aforementioned vascular disorders as the benefits of the invention are particularly manifest in the therapeutic treatment of these disorders. The present estrogen substances are distinct from both the biogenic and synthetic estrogens that are commonly applied in pharmaceutical formulations in that they contain at least 4 hydroxyl groups. The present substances are particularly special in that the 5 membered ring in the steroid skeleton comprises 3 hydroxyl substituents rather than 0-2.

Preferably, the steroid applied as the active component in the present composition is a so called biogenic estrogen, i.e. an estrogen that occurs naturally in the human body, a precursor of a biogenic estrogen or a mixture thereof. Because biogenic estrogens are naturally present in the fetal and female body, side-effects are not expected to occur, particularly not if the serum levels resulting from the exogenous administration of such estrogens do not substantially exceed naturally occurring concentrations. Naturally occurring steroids typically exhibit a 8β, 9α, 13β, 14α configuration of the steroid-skeleton.

In a preferred embodiment of the present invention the steroid contains 4 hydroxyl groups. Also, in the aforementioned formula, R₁ preferably represents a hydrogen atom. In said formula preferably at least 2, more preferably at least 3 of the groups R₁, R₂, R₃ and R₄ represent a hydrogen atom.

The steroids according to the formula encompass various enantiomers since the carbon atoms that carry hydroxyl-substituents R₅, R₆ and R₇ are chirally active. In one preferred embodiment, the present steroid is 15α-hydroxy substituted. In another preferred embodiment the substance is 16α-hydroxy substituted. In yet another preferred embodiment, the substances is 17β-hydroxy substituted. Most preferably the steroids are 15α,16α,17β-trihydroxy substituted.

In a preferred embodiment of the present invention R₃ represents a hydroxyl group or an alkoxy group. In another preferred embodiment the groups R₁, R₂ and R₄ represent hydrogen atoms, in which case, if R₃, R₅, R₆ and R₇ are hydroxyl groups, the substance is 1,3,5 (10)-estratrien-3, 15,16,17-tetrol. A preferred isomer of the latter substance is 1,3,5 (10)-estratrien-3, 15α,16α,17β-tetrol (estetrol).

Typical examples of precursors which can suitably be used in accordance with the invention are esters that can be obtained by reacting the hydroxyl groups of the estrogen substances with substances that contain one or more carboxy (M⁺ -OOC-) groups, wherein M⁺ represents a hydrogen or (akali)metal cation. Hence, in a particularly preferred embodiment, the precursors are derivatives of the estrogen substances, wherein the hydrogen atom of at least one of the hydroxyl groups in said formula has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

In a particularly preferred embodiment of the invention, the present method is used to treat an acute vascular disorder selected from the group consisting of acute cardiovascular disorders, acute cerebrovascular disorders and acute peripheral vascular disorders.

The good results obtained by the present method are deemed to be associated with vasodilator properties of the present steroid. Thus, in a preferred embodiment, said steroid is administered in an amount effective to induce vascular dilatation in the mammal.

The present method is particularly suitable for treating an acute cardiovascular disorder selected from the group consisting of angina pectoris, myocardial ischemia, myocardial infarction. The present method offers the advantage that it will induce almost instantaneous relaxation of the coronary arteries, resulting in a lowering of the blood pressure, increased blood (and oxygen supply) to myocardial tissue and decreased oxygen demand by cardiac muscles. Thus, the method is particularly suited for treating angina pectoris since it will help to restore the balance between myocardial oxygen demand and coronary oxygen supply. The increased coronary oxygen supply achieved by the present method is also beneficial in the treatment of acute myocardial ischemia and myocardial infarction resulting from said ischemia.

The present method is also very suitable for treating an acute cerebrovascular disorder selected from the group consisting of migraine, cerebral ischemia, cerebral infarction, cerebral vasospasm and thrombotic stroke. The cerebral vasodilatation achieved by the present method will increase the oxygen supply to the brain and thereby help to minimise the adverse effects of cerebral ischemia, such as resulting from cerebral infarction or cerebral vasospasm occurring after subarachnoid hemorrhage. The present method is particularly suitable for the treatment of migraine, especially if the treatment is started before the acute painful headache stage.

The phases of a migraine headache have been separated into the prodrome, aura and acute painful headache stages. The prodrome is the primary stage of a migraine attack characterised by an alteration of mood, energy or passive functions. This stage can occur for hours before the onset of the headache. The mood alterations include euphoria, loquaciousness, unprovoked apathy, depression, inertia, drowsiness, irritability, repetitive yawning, aggression and heightened sensitivity to various levels of sound (sonophobia). Nausea and vomiting, as well as paresthesias in the extremities, may also accompany these symptoms. The aura or second stage has been defined by the onset of fear of light (photophobia) and visual disturbances brought on by a reduction of cerebral blood flow due to vasospasm. The headache phase is vascular with distension and increased pulsation of cranial arteries. Increased vascular permeability is associated with the release of peptides, especially bradykinin and substance P, as well as molecules such as histamine and serotonin, which cause local pain when they reach perivascular nerve endings.

The present method can also advantageously be used in the treatment of an acute peripheral vascular disorder, particularly an acute peripheral vascular disorder selected from the group consisting of occlusive peripheral arterial disease, Buerger's disease, erectile dysfunction, Raynaud's disease, Raynaud's phenomenon and acrocyanosis.

The advantages of the present method are most pronounced if the steroid is administered orally within 15 minutes, after the mammal has started experiencing first symptoms associated with the acute vascular disorder. The rapid onset of action of the present steroid enables a fast suppression/inhibition of undesirable symptoms and consequences of the aforementioned vascular disorders. By counteracting these negative effects early on, the intensity of the ensuing symptoms (e.g. migraine headache or pain in the chest) may be suppressed effectively and tissue damage (infarction and/or ischemic reperfusion damage) may be minimised.

The method according to the invention is particularly suitable for treating a mammal that is suffering from acute symptoms associated with vasoconstriction or vascular occlusion. Both vasoconstriction and vascular occlusion will result in elevated blood pressure, limited oxygen supply and elevated oxygen demand by the cardiac muscles. As explained before, the vasodilatation induced by the present method will counteract these negative effects of vasoconstriction or vascular occlusion. The present method is particularly adequate for treating acute vasoconstriction in the brain or feelings of ill-health caused by vasoconstriction in the brain. To derive the full benefits of the present method it is advisable that the steroid be administered within 30 minutes after the mammal has started experiencing first symptoms associated with vasoconstriction.

As explained above, the present invention does not encompass the prophylactic treatment of acute vascular disorders. Instead, the present method employs on demand administration of the steroid. Thus, typically the method does not comprise administration of the steroid in accordance with a predetermined protocol, e.g. administration at regular intervals.

The present method may advantageously be employed in the treatment of humans, cattle, sheep, pigs, goat, horses as well as pets such as dogs and cats. Most preferably the present method is employed in humans. In a particularly preferred embodiment, the present method is employed in male human subjects.

The present method offers the important advantage that it allows subjects to treat themselves by orally self-administering the steroid as soon as the need arises.

Thus, the method comprises ad lib (ad libitum) self-administration of the steroid.

Good results can be obtained with the present method if the steroid is orally administered in an amount of less than 10 mg per kg of bodyweight, preferably of less than 5 mg per kg of bodyweight, more preferably of less than 2.5 mg per kg of bodyweight. In order to achieve a significant impact from the administration of the present steroid, it is advisable to orally administer in an amount of at least 2.5 µg per kg ofbodyweight. Preferably, the orally administered amount is at least 5 µg per kg of bodyweight. More preferably, the orally administered amount is at least 10 µg per kg of bodyweight.

In the present method, particularly when used in humans, the steroid is usually administered in an average dosage of at least 0.125 mg, preferably of at least 0.25 mg and more preferably of at least 0.5 mg. The maximum dosage is normally kept below 500 mg, preferably below 250 mg and more preferably below 125 mg.

In the present method, the steroid is preferably administered in an amount effective to achieve a blood serum concentration of at least 150 nanogram per litre, more preferably of at least 300 nanogram per litre, most preferably at least 600 nanogram per litre. Generally the resulting blood serum concentration of the steroid will not exceed 1200 µg per litre, preferably it will not exceed 600 µg per litre, more preferably it will not exceed 300 µg per litre.

In a specific embodiment, the steroid of the present invention is administered in combination with one or more vasoactive agents in the treatment of patients suffering from acute cardiovascular, cerebrovascular or peripheral vascular disorders. Administration of the steroid of the present invention in combination with one or more vasoactive agents may enhance the effectiveness of the treatment. In addition, such a combination may generate fewer or less pronounced side effects. The vasoactive agents for use in the oral treatment of acute cardiovascular, cerebrovascular or peripheral vascular disorders can suitably be: (1) a direct vasodilator (e.g nitroglycerin, isosorbide dinitrate, isosorbide mononitrate, nitropusside, minoxidil, papaverine HCl, isoxsuprine HCl, hydralazine), (2) an anti-platelet agent (e.g. aspirin, clopidrogel, dipyridamole, ticlodipine), (3) a thrombolytic (e.g. alteplace tPA, anistreplase, streptokinase, urokinase), (4) an anti-coagulant (e.g. heparin, low molecular weight heparins, warfarin), (5) an anti-arrhythmic (e.g. adenosine, amiodarone, disopyramide, flecainide, ibutilide, lidocaine, procainamide, sotalol), (6) an α-adrenergic agent (e.g. prazosin, indoramin, methydopa), (7) a β-adrenergic agent (e.g. atenolol, carvedilol, labetalol, metoprolol, propranolol), (8) an antilipemic agent (e.g. clofibrate, lovastatin, pravastatin, simvastatin), (9) a calcium-channel blocking agent (e.g. amlodipine, felodipine, nimodipine, verapamil), (10) an anticholinergic agent (e.g. atropine), (11) a hypotensive agent (e.g. phenoxybenzamine), (12) a renin-angiotensin system inhibitor (e.g. captopril, enalapril, lisinopril, losartan), (13) an inotopic agent (e.g. dobutamine, dopamine), (14) a diuretic (e.g. aldactone, chlorothiazide, furosemide), (15) an analgesic (e.g. morphine, diamorphine), (16) a glycoprotein IIb/IIIa receptor antagonist (e.g. abciximab), (17) a phosphodiesterase inhibitor (e.g. pentoxifylline, sildenafil), (18) a vasopressor (e.g. epinephrine) and/or a combination thereof.

In another preferred embodiment of the invention the present method comprises the co-administration of a progestogen, particularly if the method is employed in the treatment of female mammals. In order to counteract any potential negative effects of unopposed estrogen administration in the present method, it is preferred to co-administer a progestogenic component orally to inhibit estrogen stimulation of the endometrium or to administer a progestogenic component at least during a period of ten days at least every three months. Alternatively; intra-uterine administration of a progestogen (e.g. a progestogen-releasing intra-uterine device) may suitably be used to counteract any potential negative effects of unopposed estrogen administration in the present method.

In yet another specific embodiment, the steroid of the present invention is administered orally in combination with a 5-HT₁ agonist in the treatment of patients suffering from migraine. Administration of the steroid of the present invention in combination with a 5-HT₁ agonist (e.g. sumatriptan, rizatriptan, almotriptan, naratriptan, zolmitriptan, frovatriptan, eletriptan) may enhance the effectiveness of the treatment. In addition, such a combination may generate fewer or less pronounced side effects.

The present steroid can suitably be administered in any form of pharmaceutical formulation known in the art. The pharmaceutical formulation can be a solid or semi-solid dosage form such as tablets, capsules, cachets, pellets, pills, powders and granules, as well as fluid dosage forms such as solutions, emulsions and suspensions.

Another aspect of the present invention relates to a pharmaceutical composition containing:
a. at least 0.05 mg of the present steroid;
b. at least 0.01 mg of a direct vasodilator, preferably a direct vasodilator selected from the group consisting of nitroglycerin, isosorbide dinitrate, isosorbide mononitrate, nitropusside, minoxidil, papaverine, isoxsuprine and hydralazine; and
c. pharmaceutically acceptable excipient(s). The combined administration of the steroid and a direct vasodilator is particularly advantageous in the treatment of acute cardiovascular disorders.

Although it is feasible to combine the steroid and the direct vasodilator in a single pharmaceutical formulation, it may be advantageous to incorporate them into separate dosage units designed for different modes of administration. In particular, it may be advantageous to provide the steroid in an oral dosage unit and to provide the direct vasodilator in the form of a sublingual or transdermal dosage unit. Accordingly, one embodiment of the invention concerns a pharmaceutical kit comprising one or more oral dosage units containing at least 0.05 mg of the present steroid and a pharmaceutically acceptable excipient(s); and one or more sublingual or transdermal dosage units containing at least 0.01 mg of a direct vasodilator; and a pharmaceutically acceptable excipient(s).

A further aspect of the invention concerns a pharmaceutical composition containing:
a. at least 0.05 mg of the present steroid;
b. at least 0.01 mg of a 5-HT; agonist, preferably a 5-HT₁ agonist selected from the group consisting of sumatriptan, rizatriptan, almotriptan, naratriptan, zolnutriptan, frovatriptan and eletriptan; and
c. pharmaceutically acceptable excipient(s). Formulations containing the steroid in combination with a 5-HT₁ agonist produce exceptionally favourable results in the treatment of acute cerebrovascular disorders, especially migraine.

Yet another aspect of the invention relates to a pharmaceutical composition containing:
a. at least 0.05 mg of the present steroid;
b. at least 0.01 mg of a phosphodiesterase inhibitor, preferably a phosphodiesterase inhibitor selected from the group consisting of pentoxifylline and sildenafil; and
c. pharmaceutically acceptable excipient(s). Formulations containing this particular combination of active principles produce exceptionally good results in the treatment of erectile dysfunction.

The present invention also encompasses a drug delivery system comprising a pharmaceutical composition as defined above, said drug delivery system being selected from the group consisting of an oral dosage unit and a suppository. Most preferably, the drug delivery system is an oral dosage unit.

Examples of oral dosage units that may be used in accordance with the present invention include solid or semi-solid dosage forms such as tablets, capsules, cachets, pellets, pills, powders and granules. The term "solid or semi-solid dosage form" also encompasses capsules that contain a liquid, e.g. an oil, in which the present steroid is dissolved or dispersed. Tablets and equivalent solid and semi-solid dosage fouls can suitably contain materials such as binders (e.g. hydroxypropylmethyl cellulose, polyvinyl pyrrolidine, other cellulosic materials and starch), diluents (e.g. lactose and other sugars, starch, dicalcium phosphate and cellulosic materials), disintegrating agents (e.g. starch polymers and cellulosic materials) and lubricating agents (e.g., stearates and talc).

The invention is further illustrated by means of the following examples.

### EXAMPLES

### Example 1

A double-blind, randomized, placebo-controlled, single dose study was performed to determine the safety, tolerability and pharmacokinetics of 1, 10 and 100 mg orally administered estetrol in healthy postmenopausal volunteers (menopause defined as ≥ 12 months amenorrhea or six months amenorrhea with serum FSH levels ≥40 IU/L and serum E₂ <73 pmol/L), a Body Mass Index of 18-30 kg/m², non-smoking, good physical and mental health as judged by the investigator determined by medical history, physical examination, clinical laboratory, vital signs and ECG recording). In each dose group, six subjects received estetrol treatment and two subjects received placebo.

Estetrol and placebo medication was formulated as a solution (propylene glycol/water mixture) and was delivered in separate bottles for each volunteer. Bottles contained 1 mg or 10 mg estetrol per 50 ml for subjects of the groups that received 1 mg or 10 mg estetrol and 100 mg per 200 ml for the group receiving 100 mg estetrol.

Screening took place within two weeks before investigational drug administration. During and after drug administration, subjects were admitted to a Clinical Pharmacology Unit for approximately 60 hours for intensive safety monitoring. Safety and tolerability were documented by monitoring (serious) adverse events, physical examinations including vital signs, and safety laboratory assessments (hematology, biochemistry, urinalysis). Frequent blood sampling (within the range of 15 minutes to 168 hours) was performed to assess estetrol plasma concentration and pharmacokinetic variables. For this, plasma samples were subjected to liquid-liquid extraction (hexane and diethyl ether) and further subjected to HPLC analysis (Perkin Elmer 200) and tandem mass spectrometry using a PE Sciex 4000 tandem mass spectrometer and APCI interface. With each sample batch, a calibration curve with 6 calibrators was recorded. The calibration curve was calculated using linear regression (correlation coefficient > 0.98), which permitted quantification of estetrol plasma concentrations.

Evaluation of the safety and tolerability of estetrol at single oral doses of 1, 10 and 100 mg demonstrated a safe and well tolerated drug profile. The incidence of adverse events was similar in frequency and in nature on placebo and on 1, 10 and 100 mg estetrol medication. No severe or serious adverse events were reported. Furthermore, no clinically significant changes in vital signs, haematology, biochemistry or urinalysis were observed relating to the use of study medication.

The pharmacokinetic profile of estetrol after oral dosing was very consistent within each dose group and throughout the dose range tested, demonstrating good gastro-intestinal absorption and a low inter-subject variability. Plots of estetrol plasma concentration as a function of time showed a very steep increase starting directly after ingestion of the drug. Maximum plasma concentrations were observed at 15 minutes post-dosing in the 1 mg oral dose group, between 15 minutes and 30 minutes post-dosing in the 10 mg oral dose group and between 15 minutes and 1 hour post-dosing in the 100 mg group. Cₘₐₓ values after 1 mg oral estetrol dosing ranged between 0.39 and 1.2 ng/mL (median 0.52 ng/mL), after 10 mg oral estetrol dosing between 5.4 and 11.6 ng/mL (median 9.4 ng/mL) and after 100 mg oral estetrol dosing between 119 and 213 ng/mL (median 148 ng/mL).

Estetrol absorption was extremely fast and followed by redistribution to extravascular tissues, after which re-absorption to the bloodstream was evident during the first 18 hours after oral intake. Subsequently, a slow phase of final elimination of estetrol started approximately 24 hours post-dosing and during which phase estetrol was eliminated with a terminal half-life of approximately 28 hours.

These data convincingly demonstrate that estetrol administration at a single oral dose produces an extremely favourable profile in that it combines fast absorption and slow elimination kinetics. Thus, estetrol is ideally suited for on demand therapeutic application in the treatment of acute vascular disorders where a rapid onset of action and sustained activity are required.

### Example 2

Estetrol as described in this invention can be processed in the usual way for preparation of orally administered pharmaceuticals, e.g. to tablets, dragees, capsules, pills, suspensions, or solutions. The pharmaceuticals of the invention can be used for treating acute cardiovascular disorders such as angina pectoris induced by coronary artery disease.

A clinical trial is conducted with single dose, on demand oral estetrol administration of 100 mg in subjects suffering from documented angina pectoris and is compared to placebo treatment. Additionally, patients in this trial are offered to use their standard on demand medication, e.g. nitroglycerin. Hundred patients, males and females, with a diagnosis of angina pectoris induced by coronary artery disease, substantiated by positive dynamic tests of myocardial ischaemia, participate in the clinical trial. Patients are selected according to the following inclusion (I) and exclusion (E) criteria: Patients with clear angina pectoris diagnosis (I), patients having chest pain attacks more than two times a week (I). Patients with active myocardial infarct or other cardiopulmonary heart disease, such as severe psychoneurosis, menopausal syndrome and chest pain caused by cervical spondylopathy (E), patients of angina pectoris combined with hypertension (bloodpressure >24/14.87 Kpa) (E), patients of severe cardiopulmonary function deficiency, severe arrhythmia, abnormal liver and renal function and/or hematopoietic disease (E), breast-feeding women and patients with a known history of allergic disease (E), patients younger than 18 or older than 70 (E).

The diagnostic standard for angina pectoris severity is used to describe baseline patient characteristics and subsequently to monitor treatment efficacy (see below). Low-grade: Typical attack of angina pectoris, which usually lasts a few minutes and occurs 1-3 times a day. The attack is not severe, but the patient sometimes needs to take nitroglycerin.

Mid-grade: Angina attack occurs more than 3 times everyday and lasts several to ten minutes every time. The pain is more severe than that of the low-grade attack and the patient generally needs sublingual nitroglycerin. High-grade: Angina attack occurs frequently daily. It lasts longer and more seriously. Routine life is affected. The patient needs to use nitroglycerin frequently.

The diagnostic standard for angina pectoris types is used to describe baseline characteristics and classify patient types.
Exertionally-induced angina pectoris: The transient attack of chest pain is caused typically by exertion or other conditions that increase myocardial oxygen demands. The pain normally disappears rapidly after rest or administration of sublingual nitroglycerin.
Spontaneous angina pectoris: The attack of chest pain is not obviously related to increase of myocardial oxygen consumption. Compared with tiredness-induced angina pectoris, the pain lasts longer and is more severe. It can not be easily alleviated by nitroglycerin.
Mixed Angina Pectoris: Spontaneous angina pectoris can come with tiredness-induced angina pectoris and is classified as mixed angina pectoris.

After establishing baseline characteristics patients are randomized for age, sex, duration of illness, type and seriousness of angina pectoris to receive on demand oral estetrol treatment with 100 mg per dose (n=50) or oral treatment with placebo medication (n=50). Subjects are instructed on the use of investigational drug medication, which is preferentially being taken instantly at the moment when the first signs of angina pectoris appear. Additionally, subjects are offered to choose nitroglycerin as an alternative mode of on demand medication. Patients are asked to keep daily records stating the number of attacks, severity of attacks, duration of attacks and use of medication (investigational drug or nitroglycerin) for the entire study period of 4 weeks. Ill-compliance drop-outs are replaced if they do not take the medication as prescribed or if the record forms are filled out incompletely.

For patients in the estetrol arm of the study no obvious side effects are noted during the clinical trial period differing significantly from patients taking the placebo medication. The use of nitroglycerin in the estetrol arm of the study is significantly lower than in the placebo arm of the study. Patients report relief of angina pectoris symptoms after taking estetrol medication and are less prone than placebo-receiving patients to change from investigational drug medication to nitroglycerin. Overall, patients in the estetrol arm report statistically more improvement of exercise tolerance, ability to extend the duration of walking and quality of life. Interestingly, patients taking estetrol medication report that concomitant use of estetrol and nitroglycerin provided them with a more rapid decline of angina pectoris symptoms, particularly during severe attacks, then use of either estetrol or nitroglycerin alone.

It is concluded that on demand estetrol medication is safe and effective in treating angina pectoris due to coronary artery disease. Furthermore, it is concluded that the combination of on demand estetrol medication with conventional medication, e.g. nitroglycerin, acts synergistically on alleviating (severe) symptoms of angina pectoris.

### Example 3

A double-blind, placebo-controlled, multiple attack clinical study is conducted with single dose, on demand oral estetrol administration in adult volunteers (20 females and 20 males) each of whom is seeking migraine relief.

Participating subjects show at least a 1-year history of migraine as is defined by International Headache Society criteria (IHS; Headache Classification Committee of the International Headache Society, 1988, Cephalalgia 8(Suppl. 7):19-28) and report regularly occurring migraines with episodes of pain, photophobia and nausea. Each participant receives blinded medication in two differently labeled bottles, filled with tablets. One bottle comprises tablets that contain 100 mg estetrol, whilst the other bottle contains identical placebo tablets. Participants are allowed to choose from which bottle to orally ingest a tablet at the moment the first symptoms of a migraine attack become apparent. Patients are asked to keep daily records stating the number of migraine attacks, severity of attacks, duration of attacks and use of medication (bottle 1 or bottle 2) for the entire study period of 2 months. Patients are dropped from the study and are replaced if they do not take the medication as prescribed or if the record forms are filled out incompletely.

No apparent side effects are noted during the clinical trial period which are directly related to the investigational drug medication. It is found that during the study participants use significantly more estetrol containing tablets than placebo tablets. In addition, analysis of the data shows that participants who are using estetrol containing tablets report less headache and more quicker relief of migraine symptoms than they have themselves reported at baseline for the use of analgesic and/or triptan-type medication. '

It is concluded that on demand estetrol medication as is described in this invention is safe and effective in treating migraine.

### Example 4

An adult female with an established history of suffering from migraine attacks complains of a migraine attack consisting of typical migraine headache, nausea and sensitivity to light and sound. She is dosed with a single oral tablet containing 10 mg sumatriptan and 100 mg estetrol. Her symptoms start to diminish within 30 minutes. After two hours she is completely symptom free and has no relapse over the next 48 hours. Her pain is relieved more quickly and with longer uninterrupted relief than when she takes sumatriptan alone at an oral dose of 10 mg. She experiences fewer adverse sumatriptan drug reactions than she reports having experienced previously on recommended sumatriptan doses of 25-100 mg, with particular reference to asthenia and flushing.

### Example 5

An adult male exhibits the same history and indication as in example 4. Treatment is by means of a single oral tablet containing 10 mg sumatriptan and 50 mg estetrol. The same result as in example 4 is obtained.

### Example 6

In contrast to subjects suffering from erectile dysfunction due to hormonal insufficiency and whom can be suitably treated with androgen substitution therapy, there is no such current standard treatment to overcome impotence in men with vascular deficits. A current treatment option encompasses the oral administration of sildenafil. However, sildenafil is contraindicated in subjects who are allergic to sildenafil, or, due to vascular problems, are concurrently being treated with a nitrate medicine (e.g nitroglycerin, a transdermal nitrate, isosorbide nitrate), or an anti-infective such as erythromycin, ketoconazole, itraconazole, or rifampin. Other non-oral and non-steroid treatments for erectile dysfunction include the use of intracavemous injections consisting of direct injection of vasodilatory drugs, e.g injections with papaverine, or phentolamine into the corpora cavemosa of the penis (Campell's Urology, ed. W.B. Saunders Company, 6.sup.th Edition, Volume III, 3055-3057). Although intracavemous injection is efficient and scientifically approved, it has serious disadvantages mainly relating to the form (injection) as well as route of administration (intracarvernosal).

As a novel method single dose, on demand oral estetrol administration is tested to treat erectile dysfunction and compared to placebo treatment. A clinical trial is performed in 20 subjects, experiencing erectile dysfunction that is primarily due to vascular causes and in whom intracavemous injection of either papaverine or phentolamine has been previously proven successful in restoring erectile function. Ten subjects receive estetrol medication (100 mg per dose) and 10 subjects receive placebo medication in this outclinic study.

Subjects with mild, moderate as well as severe symptoms of erectile dysfunction are instructed on the use of investigational drug medication, which is preferentially being taken 15 minutes to 2 hours before sexual activity. At the start of the trial, subjects are asked to fill out a baseline Sexual Functioning Questionnaire (SFQ). The SFQ is used as the primary endpoint, since this is a self-administered questionnaire and designed to measure changes in sexual function (Smith et al., 2002, British J. Psych., 181:49-55). It asks detailed questions about libido, physical arousal (erection), masturbation, orgasm and ejaculation. It is designed such that higher scores indicate greater dysfunction. Typically total normal controls scores are less than 14 and sexually disfunctioning patients show total scores of more than 18 (Smith et al., 2002, British J. Psych., 181:49-55).

During the study estetrol is well tolerated. At the end of the study period at 4 weeks, subject's responses to SFQ survey questions are compared to SFQ responses at baseline. For separate SFQ variables, a comparison is made of those subjects who initially show a bad baseline score. The results are then pooled and expressed as the percentage of subjects with improvement. Single dose, on demand administration of estetrol results in a higher percentage of subjects that show improvement in the ability to develop and maintain erections as compared to subjects receiving placebo treatment. Also more subjects report improvement in satisfaction with their overall sex life when taking estetrol as compared to placebo medication.

These results demonstrate a positive pharmacological outcome of estetrol in the treatment of men suffering from erectile dysfunction. The results further demonstrate the rapid onset of action of orally administered estetrol and a favourable side effect profile. Oral administration of estetrol therefore represents an effective treatment modality with advantages over current classes of drugs in the treatment of erectile dysfunction.

### Example 7

Preparation of 100 capsules, containing 100 mg estetrol and 25 mg sildenafil citrate per capsule: 10.0 gram estetrol and 2.5 gram sildenafil citrate are mixed together. The resulting powder mixture is transferred into a beaker with volume indication. Volume is adjusted to 46.5 ml with Primogel® (sodium starch glycolate). Subsequently, the powder mixture is filled into 100 two-piece hard gelatine capsules (Capsugel® 1).

## Claims

1. Use of a steroid in the manufacture of a pharmaceutical composition for use in a method of treating an acute vascular disorder in a mammal, said method comprising ad lib oral administration to said mammal, upon demand, of an effective amount of the steroid to the mammal, said steroid being selected from the group consisting of:
substances represented by the following formula 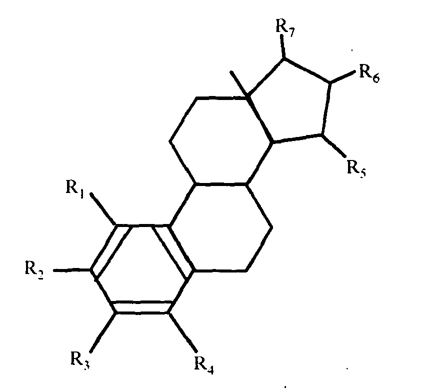 in which formula R₁, R₂, R₃, R₄ independently are a hydrogen atom, a hydroxyl group or an alkoxy group with 1-5 carbon atoms; each of R₅, R₆, R₇ is a hydroxyl group; no more than 3 of R₁, R₂, R₃, R₄ are hydrogen atoms;
precursors capable of liberating a substance according to the aforementioned formula when used in the present method, which precursors are derivatives of the aforementioned steroids wherein the hydrogen atom of at least one of the hydroxyl groups has been substituted by an acyl radical of a hydrocarbon carboxylic, sulfonic or sulfamic acid of 1-25 carbon atoms; tetrahydrofuranyl; tetrahydropyranal; or a straight or branched chain glycosidic residue containing 1-20 glycosidic units per residue; and mixtures of one or more of the aforementioned substances and/or precursors, wherein the steroid is administered within 30 minutes after the mammal has started experiencing first symptoms associated with the acute vascular disorder.

2. Use according to claim 1, wherein R₃ represent a hydroxyl group or an alkoxy group.

3. Use according to claim 1 or 2, wherein at least 3 of the groups R₁, R₂, R₃ and R₄ represent hydrogen atoms.

4. Use according to any one of the preceding claims, wherein the acute vascular disorder is selected from the group consisting of acute cardiovascular disorders, acute cerebrovascular disorders and acute peripheral vascular disorders.

5. Use according to claim 4, wherein the acute cardiovascular disorder is angina pectoris.

6. Use according to claim 4, wherein the acute cerebrovascular disorder is migraine.

7. Use according to any one of the preceding claims, wherein the steroid is administered within 15 minutes after the mammal has started experiencing first symptoms associated with the acute vascular disorder.

8. Use according to any one of the preceding claims, wherein the method does not comprises administration of the steroid at regular intervals.

9. Use according to any one of the preceding claims, wherein the steroid is administered in a dosage of at least 2.5 µg per kg of bodyweight, preferably of at least 5 µg per kg of bodyweight.

10. A pharmaceutical composition containing:
a. at least 0.05 mg of a steroid as defined in claim 1;
b. at least 0.01 mg of a direct vasodilator, preferably a direct vasodilator selected from the group consisting of nitroglycerin, isosorbide dinitrate, isosorbide mononitrate, nitropusside, minoxidil, papaverine, isoxsuprinc and hydralazine; and
c. pharmaceutically acceptable exciplent(s).

11. A pharmaceutical composition containing:
a. at least 0.05 mg of a steroid as defined in claim 1 ;
b. at least 0.01 mg of a 5-HT₁ agonist, preferably a 5-HT₁ agonist selected from the group consisting of sumatriptan, rizatriptan, almotriptan, naratriptan, zolmitriptan, frovatriptan and eletriptan; and
c. pharmaceutically acceptable excipient(s).

12. A pharmaceutical composition containing:
a. at least 0.05 mg of a steroid as defined in claim 1;
b. at least 0.01 mg of a phosphodiesterase inhibitor, preferably a phosphodiesterase inhibitor selected from the group consisting of pentoxifylline and sildenafil; and
c. pharmaceutically acceptable excipient(s).

13. A drug delivery system comprising a pharmaceutical composition according to any one of claims 10-12, said drug delivery system being delivered orally.

14. A pharmaceutical kit comprising one or more oral dosage units containing at least 0.05 mg of the steroid as defined in claim 1 and a pharmaceutically acceptable excipient(s); and one or more sublingual or transdermal dosage units containing at least 0.01 mg of a direct vasodilator, preferably a direct vasodilator selected from the group consisting of nitroglycerin, isosorbide dinitrate, isosorbide mononitrate, nitropusside, minoxidil, papaverine, isoxsuprine and hydralazine; and a pharmaceutically acceptable excipient.

## Patentansprüche

1. Verwendung eines Steroids zur Herstellung einer pharmazeutischen Zusammensetzung zur Verwendung in einem Verfahren zur Behandlung einer akuten Gefässerkrankung in einem Säuger, wobei das Verfahren eine bedart bedingte orale ad lib-Verabreichung einer für den Säuger wirksamen Menge des Steroids an den Säuger umfasst, wobei das Steroid aus der Gruppe ausgewählt wird, bestehend aus: Substanzen, die durch die folgende Formel dargestellt werden 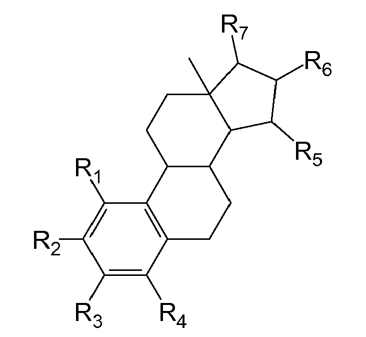 worin R₁, R₂, R₃ und R₄ unabhängig voneinander ein Wasserstoffatom, eine Hydroxylgruppe oder eine Alkoxygruppe mit 1 bis 5 Kohlenstoffatomen sind; jedes von R₅, R₆ und R₇ eine Hydroxylgruppe ist; nicht mehr als drei von R₁, R₂, R₃ und R₄ Wasserstoffatome sind;
Vorläufern, die geeignet sind, eine Substanz gemäss der zuvor erwähnten Formel freizusetzen, wenn sie in dem vorliegenden Verfahren verwendet werden, wobei die Vorläufer Derivate der zuvor erwähnten Steroide sind, worin das Wasserstoffatom von mindestens einer der Hydroxylgruppen durch ein Acylradikal einer Kohlenwasserstoffcarbon-, -sulfon- oder -sulfamsäure mit 1 bis 25 Kohlenstoffatomen; Tetrahydrofuranyl; Tetrahydropyranyl oder einem geradkettigen oder verzweigten glycosidischen Rest mit 1 bis 20 glycosidischen Einheiten pro Rest substituiert wurde; und Mischungen von eine(r/m) oder mehreren der zuvor erwähnten Substanzen und/oder Vorläufer, wobei das Steroid innerhalb von 30 Minuten verabreicht wird, nachdem der Säuger begonnen hat, erste Symptome, die mit der akuten Gefässerkrankung verbunden sind, zu empfinden.

2. Verwendung gemäss Anspruch 1, wobei R₃ eine Hydroxylgruppe oder eine Alkoxygruppe darstellt.

3. Verwendung gemäss Anspruch 1 oder 2, wobei mindestens drei der Gruppen R₁, R₂, R₃ und R₄ Wasserstoffatome darstellen.

4. Verwendung gemäss mindestens einem der vorhergehenden Ansprüche, wobei die akute Gefässerkrankung aus der Gruppe ausgewählt wird, die aus akuten kardiovaskulären Erkrankungen, akuten cerebrovaskulären Erkrankungen und akuten peripheren Gefässerkrankungen besteht.

5. Verwendung gemäss Anspruch 4, wobei die akute kardiovaskuläre Erkrankung Angina pectoris ist.

6. Verwendung gemäss Anspruch 4, wobei die akute cerebrovaskuläre Erkrankung Migräne ist.

7. Verwendung gemäss mindestens einem der vorhergehenden Ansprüche, wobei das Steroid innerhalb von 15 Minuten verabreicht wird, nachdem der Säuger begonnen hat, erste Symptome, die mit der akuten Gefässerkrankung verbunden sind, zu empfinden.

8. Verwendung gemäss mindestens einem der vorhergehenden Ansprüche, wobei das Verfahren keine Verabreichung des Steroids in regelmässigen Abständen umfasst.

9. Verwendung gemäss mindestens einem der vorhergehenden Ansprüche, wobei das Steroid in einer Dosierung von mindestens 2,5 µg pro kg Körpergewicht, vorzugsweise mindestens 5 µg pro kg Körpergewicht, verabreicht wird.

10. Pharmazeutische Zusammensetzung, enthaltend:
(a) mindestens 0,05 mg eines wie in Anspruch 1 definierten Steroids;
(b) mindestens 0,01 mg eines direkten Vasodilators, vorzugsweise eines direkten Vasodilators, ausgewählt aus der Gruppe bestehend aus Nitroglycerin, Isosorbiddinitrat, Isosorbidmononitrat, Nitropussid, Minoxidil, Papaverin, Isoxsuprin und Hydralazin; und
(c) (einen) pharmazeutisch akzeptable (n) Hilfsstoff(e).

11. Pharmazeutische Zusammensetzung, enthaltend:
(a) mindestens 0,05 mg eines wie in Anspruch 1 definierten Steroids;
(b) mindestens 0,01 mg eines 5-HT₁-Agonisten, vorzugsweise eines 5-HT₁-Agonisten, ausgewählt aus der Gruppe bestehend aus Sumatriptan, Rizatriptan, Almotriptan, Naratriptan, Zolmitriptan, Frovatriptan und Eletriptan; und
(c) (einen) pharmazeutisch akzeptable (n) Hilfsstoff(e).

12. Pharmazeutische Zusammensetzung, enthaltend:
(a) mindestens 0,05 mg eines wie in Anspruch 1 definierten Steroids;
(b) mindestens 0,01 mg eines Phosphodiesteraseinhibitors, vorzugsweise eines Phosphodiesteraseinhibitors, ausgewählt aus der Gruppe bestehend aus Pentoxyfyllin und Sildenafil; und
(c) (einen) pharmazeutisch akzeptable (n) Hilfsstoff (e) .

13. Arzneimittel-Verabreichungssystem, umfassend eine pharmazeutische Zusammensetzung gemäss mindestens einem der Ansprüche 10 bis 12, wobei das Arzneimittel-Verabreichungssystem oral verabreicht wird.

14. Pharmazeutischer Kit, umfassend eine oder mehrere orale Dosierungseinheiten, die mindestens 0,05 mg des in Anspruch 1 definierten Steroids und(einen) pharmazeutisch akzeptable(n) Hilfsstoff(e) enthält; und eine oder mehrere sublinguale oder transdermale Dosierungseinheiten, die mindestens 0,01 mg eines direkten Vasodilators, vorzugsweise eines direkten Vasodilators, ausgewählt aus der Gruppe bestehend aus Nitroglycerin, Isosorbiddinitrat, Isosorbidmononitrat, Nitropussid, Minoxidil, Papaverin, Isoxsuprin und Hydralazin; und (einen) pharmazeutisch akzeptable(n) Hilfsstoff(e) enthalten.

## Revendications

1. Utilisation d'un stéroïde pour la préparation d'une composition pharmaceutique destinée à être utilisée dans un procédé de traitement d'un trouble vasculaire aiguë chez un mammifère, selon lequel on administre à volonté par voie orale à ce mammifère, sur demande, une quantité efficace de stéroïde, ce stéroïde étant choisi dans le groupe formé par les composés représentés par la formule ci-dessous : 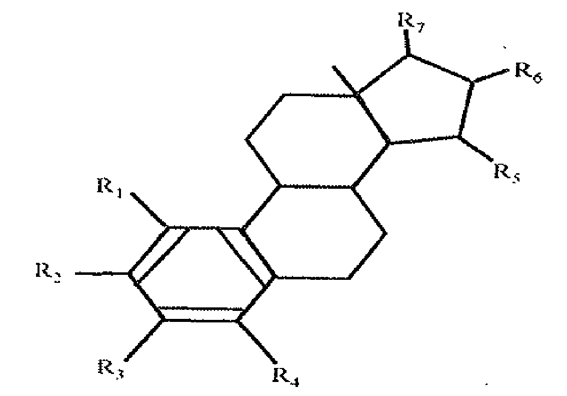 dans laquelle R₁, R₂, R₃, R₄ représentent indépendamment un atome d'hydrogène, un groupe hydroxyle ou un groupe alkoxy ayant 1 à 5 atomes de carbone, R₅, R₆, R₇ représentent chacun un groupe hydroxyle, et pas plus de 3, R₁, R₂, R₃, R₄ sont des atomes d'hydrogène ; de précurseurs susceptibles de libérer un composé selon la formule ci-dessus lorsqu'ils sont utilisés dans ce procédé, ces précurseurs étant des dérivés des stéroïdes susmentionnés, l'atome d'hydrogène d'au moins l'un des groupes hydroxyle ayant été substitué par un radical acyle d'un acide carboxylique, sulfonique ou sulfamique hydrocarboné ayant 1-25 atomes de carbone, tétrahydrofuranyl, tétrahydropyranal, ou un résidu glycosidique à chaine droite ou ramifiée renfermant 1-20 unités glycosidique par résidu, et de mélanges d'au moins l'un des composés et/ou précurseurs susmentionnés, le stéroïde étant administré dans les 30 minutes après que le mammifère ait commencé à éprouver les premiers symptômes associés au trouble vasculaire aiguë.

2. Utilisation conforme à la revendication 1, selon laquelle R₃ représente un groupe hydroxyle ou un groupe alkoxy.

3. Utilisation conforme à la revendication 1 ou à la revendication 2, selon laquelle au moins 3 des groupes R₁, R₂, R₃ et R₄ représentent des atomes d'hydrogène.

4. Utilisation conforme à l'une quelconque des revendications précédentes, selon laquelle le trouble vasculaire aiguë est choisi dans le groupe formé par les troubles cardio-vasculaires aiguës, les troubles cérébro-vasculaires aiguës et les troubles vasculaires périphériques aiguës.

5. Utilisation conforme à la revendication 4, selon laquelle le trouble cardio-vasculaire aiguë est l'angine de poitrine.

6. Utilisation conforme à la revendication 4, selon laquelle le trouble cérébro-vasculaire aiguë est la migraine.

7. Utilisation conforme à l'une quelconques des revendications précédentes, selon laquelle le stéroïde est administré dans les 15 minutes après que le mammifère ait commencé à éprouver les premiers symptômes associés au trouble vasculaire aiguë.

8. Utilisation conforme à l'une quelconque des revendications précédentes, selon lequel le procédé ne consiste pas à administrer le stéroïde à des intervalles réguliers.

9. Utilisation conforme à l'une quelconque des revendications précédentes, selon laquelle le stéroïde est administré selon une posologie d'au moins 2,5 µg par kilogramme de poids corporel, de préférence d'au moins 5 µg par kilogramme de poids corporel.

10. Composition pharmaceutique renfermant :
a. au moins 0,05 mg d'un stéroïde conforme à la revendication 1,
b. au moins 0,01 mg d'un vasodilateur direct de préférence un vaso dilatateur direct choisi dans le groupe formé par la nitroglycérine, le dinitrate d'isosorbide, le mononitrate d'isosorbide, le nitroprussiate, le minoxidil, la papavérine, l'isoxsuprine et l'hydralazine, et
c. au moins un excipient pharmacologiquement acceptable.

11. Composition pharmaceutique renfermant :
a. au moins 0,05 mg d'un stéroïde conforme à la revendication 1,
b. au moins 0,01 mg d'un agoniste 5-HT₁, de préférence d'un agoniste 5-HT₁ choisi dans le groupe formé par le sumatriptan, le rizatriptan, l'almotriptan, le naratriptan, le zolmitriptan, le frovatriptan et l'élétriptan, et
c. au moins un excipient pharmacologiquement acceptable

12. Composition pharmaceutique renfermant :
a. au moins 0,05 mg d'un stéroïde conforme à la revendication 1,
b. au moins 0,01 mg d'un inhibiteur de la phosphodiestérase, de préférence d'un inhibiteur de la phosphodiestérase choisi dans le groupe formé par la pentoxifylline et le sildénafil, et
c. au moins un excipient pharmacologiquement acceptable

13. Système d'administration d'un médicament comprenant une composition pharmaceutique selon l'une quelconque des revendications 10 à 12, ce système étant administré par voie orale.

14. Kit pharmaceutique comportant au moins une unité de posologie orale renfermant au moins 0,05 mg du stéroïde conforme à la revendication 1 et au moins un excipient pharmacologiquement acceptable ; et au moins une unité de posologie sublinguale ou transdermique renfermant au moins 0,01 mg d'un vasodilatateur direct, de préférence d'un vasodilateur direct choisi dans le groupe formé par la la nitroglycérine, le dinitrate d'isosorbide, le mononitrate d'isosorbide, le nitroprussiate, le minoxidil, la papavérine, l'isoxsuprine et l'hydralazine, et un excipient pharmacologiquement acceptable.
